Europäisches Patentamt

European Patent Office

Office européen des brevets

(11) Publication number: 0 360 619
A2

## (12) EUROPEAN PATENT APPLICATION

(21) Application number: 89309678.4

(22) Date of filing: 22.09.89

(51) Int. Cl.⁵: **C 07 C 229/26**
C 07 C 279/14,
C 07 D 233/64,
A 61 K 31/195, A 61 K 33/24,
A 61 K 31/42, C 01 G 41/02

(30) Priority: 22.09.88 US 247641

(43) Date of publication of application:
28.03.90 Bulletin 90/13

(84) Designated Contracting States:
AT BE CH DE ES FR GB GR IT LI LU NL SE

(71) Applicant: JOHNSON MATTHEY PUBLIC LIMITED
COMPANY
New Garden House 78 Hatton Garden
London, EC1N 8JP (GB)

Hill, Craig L.
2941 Cravey Drive
Atlanta, GA 30345 (US)

Schinazi, Raymond F.
Shadow Walk Lane
Tucker, GA 30084 (US)

(72) Inventor: Hill, Craig L.
2941 Cravey Drive
Atlanta, GA 30345 (US)

Schinazi, Raymond F.
Shadow Walk Lane
Tucker, GA30084 (US)

(74) Representative: Wishart, Ian Carmichael et al
Patents Department Johnson Matthey Technology
Centre Blounts Court Sonning Common
Reading, Berks RG4 9NH (GB)

(54) Antiviral agents.

(57) Polyoxometallate compounds which exhibit anti-retroviral activity, are disclosed. These compounds have the following general molecular formulas:

$(X^+)_n(H^+)_{4-n}(SiW_{12}O_{40})^{4-}$   $n = 0 - 4$

$(X^+)_n(H^+)_{5-m}(BW_{12}O_{40})^{5-}$   $m = 0 - 5$

$(X^+)_n(H^+)_{4-n}(W_{10}O_{32})^{4-}$   $n = 0 - 4$

$(X^+)_p(H^+)_{3-p}(PW_{12}O_{40})^{3-}$   $p = 0 - 3$

wherein X = K, Na, NH₄, histidineH, lysineH, arginineH, and hydrates thereof. X can generally be any naturally occurring monocationic amino acid or polypeptide containing arginine, histidine or lysine.

EP 0 360 619 A2

Description

## ANTIVIRAL AGENTS

## BACKGROUND OF THE INVENTION

Field of the Invention:

This invention relates to certain polyoxometallate compounds, particularly amino acid salts of polyoxometallate compounds, as antiviral agents. In particular, these agents can be used for treatment of infection by retroviruses such as HIV (HTLV-III/LAV), which causes acquired immunodeficiency syndrome (AIDS). The invention is also directed to a method of treatment of viral diseases, such as AIDS, which involves treating a mammal infected with HIV with a composition including one or more of the compounds of the present invention. Other retroviruses may also be treatable with the compounds of this invention.

Discussion of the Background Art:

AIDS was first recognized as early as 1979. The number of cases reported to the Centers for Disease Control (CDC) increased dramatically each year since then, and in 1982 the CDC declared AIDS a new epidemic. Over 3,000 new cases were reported in 1984 alone. As of June 1, 1988, the World Health Organization (WHO) announced that nearly 100,000 cases of AIDS worldwide had been reported. However, in reality, the WHO estimates about 200,000 cases of the disease have occurred worldwide. It has also been estimated that approximately 5,000,000 people are infected today with HIV and at least 1,000,000 new cases of AIDS may be expected during the next 5 years.

In the United States, nearly 70,000 cases of AIDS have been reported to the U.S. Centers for Disease Control (CDC). Nearly 28,000 were reported in 1987 alone. It has been predicted that in the United States 365,000 to 380,000 total cases will be reported by 1992. The CDC believes that 1 million to 1.5 million Americans are now infected with HIV. It is clear that the cost of the AIDS epidemic in terms of human lives is staggering, and the worst is yet to come.

Retroviruses were proposed as the causative agent of AIDS. Two such retroviruses now known to cause AIDS have been identified and isolated: LAV (lymphadenopathy-associated virus) and HTLV-III (human T-cell leukemia virus). It was later determined that LAV and HTLV-III are identical. Recently, human immunodeficiency virus type 1 (HIV) has emerged as a preferred name for the virus responsible for AIDS. Antibodies to HIV are present in over 80% of patients diagnosed as having AIDS or pre-AIDS syndrome, and it has also been found with high frequency in identified risk groups.

There is considerable difficulty in diagnosing the risk of development of AIDS. AIDS is known to develop in at least 10% of the individuals infected with HIV, although this percentage is suspected to be higher.

A patient is generally diagnosed as having AIDS when a previously healthy adult with an intact immune system acquires impaired T-cell immunity. The impaired immunity usually appears over a period of 18 months to 3 years. As a result of this impaired immunity, the patient becomes susceptible to opportunistic infections, various types of cancers such as Kaposi's sarcoma, and other disorders associated with reduced functioning of the immune system.

Another condition associated with HIV is AIDS-related complex, or ARC. This condition can lead to AIDS in some cases.

No treatment capable of preventing the disease or reversing the immunodeficiency of AIDS or ARC is currently available. All patients with opportunistic infections and approximately half of all patients with Kaposi's sarcoma have died within two years of diagnosis. Attempts at reviving the immune systems in patients with AIDS have been unsuccessful.

Recently, it has been reported that 3'-azido-3'-deoxythymidine (AZT) is an antiviral agent that inhibits the infectivity and cytopathic effect of HIV-1 in vitro and prolongs life in vivo. See Mitsuya, et al, Proc. Natl. Acad. Sci. USA 82, 7096-100 (1985), Fischl, et al., New Engl. J. Med. However, preliminary results indicate that this compound exhibits bone marrow toxicity and neurotoxicity in a clinical setting. See Yarchoan et al, Lancet 575-580 (1986). Richman, et al. AZT was originally synthesized by Horwitz et al., J. Org. Chem. 29, 2076-2078, 1964. Its activity against Friend leukemia virus (a retrovirus) was reported as early as 1973 (see Ostertag et al, Proc. Natl. Acad. Sci. USA 71, 4980-4985 (1974) and Krieg et al, Exptl. Cell. Res. 116, 21-29, 1978 and references cited therein). The compounds of this invention are structurally unrelated to AZT.

U.S. Patent 4,681,933 is directed to certain 2',3'-dideoxy-5-substituted uridines and related compounds as antiviral agents. Structurally, these compounds are similar to AZT, and are remote from those of the present invention.

U.S. Patent 4,759,929 is of particular relevance to the present application. This patent discloses a method of treating HIV-1 infection in warm blooded animals which involves treatment with a salt of 9-antimonio-III-21-tungsten-VI-sodate, $(NH_4^+)_{17}H^+[NaSb_9W_{21}O_{86}]$ (also known as HPA-23). This compound is a polyoxometallate, but it is compositionally different from the compounds of the present invention. Moreover, there are disadvantages associated with this drug, such as 1) it induces thrombocytopenia and displays, in general, a

2

high toxicity to human bone marrow cells, and 2) it must be taken intravenously as it degrades at pH <4.

Polyoxometallate compounds have also been discovered to have anticoagulant activity. French Patent 2,587,214 is directed to anticoagulant tungstic heteropoly anions for use in vivo and in vitro to prevent blood coagulation.

Polyoxometallates or polyoxoanions are condensed oligomeric aggregates of metal and oxygen atoms that occur in two generic forms: those that contain only these atoms (isopolyoxo anions, isopoly compounds, or isopolyoxometallates), and those that contain one or more "heteroatoms" in addition to the metal and oxygen atoms (heteropoly anions, heteropoly compounds, or heteropolyoxometallates). The heteroatoms in the latter class of complexes can be either main group ions, such as $Si^{4+}$ (as in the silicotungstate = ST), $P^{5+}$ (as in the Dawson tungstate, DT), or transition group ions, such as $Fe^{3+}$ or $Co^{2+}$. Polyoxometallates have been known for over a century, but only recently has interest in these materials increased. To some degree, this is due to the fact that these materials have become more chemically well defined. In addition, since 1977, the polyoxometallates and particularly the heteropoly compounds or heteropoly acids have received increasing attention as reagents or catalysts for redox processes involving organic substrates.

In spite of the known agents for treatment of AIDS, there continues to remain a need for new and effective methods for treatment of retroviral infections, particularly HIV infection including AIDS and AIDS related complex (ARC). It was in this context that the present invention was accomplished.

## SUMMARY OF THE INVENTION

Accordingly, it is an object of the present invention to provide new and effective compositions for treatment of viral infections, particularly retroviral infections;

It is yet another object of the present invention to provide methods for treatment of viral infections, particularly retroviral infections;

It is another object of the present invention to provide compounds and compositions for treatment of AIDS or ARC in mammals, including humans.

These and other objects of the present invention as will hereinafter become more readily apparent, have been accomplished by the discovery that polyoxometallates having the structures identified hereinbelow exert inhibitory activity towards cells infected with various retroviruses, leading to the expectation that these agents will have in vivo activity towards these retroviruses in mammals, such as humans.

## BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:

Figure 1 shows three representative polyoxometallate structural types.

Figure 2 shows a synthetic scheme for ArgH-ST, the argininium salt of silicotungstate.

Figure 3 shows the relative effect of HPA-23, ST, BT, $NH_4BT$ and $NH_4ST$ on colony formation of human granulocyte-macrophage precursor cells.

## DETAILED DESCRIPTION OF THE PREFERRED EMBODIMENTS

Polyoxometallates of the Present Invention

The polyoxometallates of the present invention may be described by the following generalized formulas:

$(X^+)_n(H^+)_{4-n}(SiW_{12}O_{40})^{4-}$    n = 0 - 4

$(X^+)_m(H^+)_{5-m}(BW_{12}O_{40})^{5-}$    m = 0 - 5

$(X^+)_n(H^+)_{4-n}(W_{10}O_{32})^{4-}$    n = 0 - 4

$(X^+)_p(H^+)_{6-p}(P_2W_{18}O_{62})^{6-}$    p = 0 - 6

wherein X = K, Na, $NH_4$, histidineH, lysineH, arginineH, and hydrates thereof. X can also be any naturally occurring monocationic amino acid or polypeptide (e.g. 2-10 amino acids, preferably 2-4 amino acids, containing one naturally occurring basic amino acid = arg, his, lys, in its protonated hence cationic form).

For the sake of simplicity and clarity, the following abbreviations will be used herein:

$(SiW_{12}O_{40})^{4-} = ST$

$(BW_{12}O_{40})^{5-} = BT$

$(W_{10}O_{32})^{4-} = W10$

$(P_2W_{18}O_{62})^{6-} = DT$

histidine = His

lysine = Lys

arginine = Arg

Preferred X groups for the present invention are the naturally occurring protonated basic amino acids histidineH, lysineH and arginineH, and in particular arginineH. However, it is also possible that other naturally occurring amino acids or small peptides which are capable of forming a positive charge under acidic conditions can be useful as cations for purposes of the present invention. Examples of the small peptides are Gly-Arg, Ala-His-Ala, and the like. The small peptides themselves may not occur naturally, but will preferably be based on naturally-occurring amino acids. Naturally occurring amino acids are generally selected from the 20 naturally-occurring amino acids which are most commonly found in proteins.

The compounds of the present invention may be synthesized by methods known to those of ordinary skill in the art. Both isopolyoxometallates, such as W10, and heteropolyoxometallates, such as ST, are most often made by carefully acidifying the correct concentrations of monomeric tungstate, usually starting with the sodium salt, $NaWO_4$, in aqueous solution, with a water-soluble precursor of the heteroatom, if applicable, followed by precipitation of the product. The appropriate water-soluble salt of the desired cations, $X^+$, is added in the later step. In the Example section herein, procedures for the preparation of ST, BT and some of the basic amino acid salts of ST and BT are provided.

Specific examples of compounds which fall within the scope of the present invention are the following:

$(NH_4)H_3ST$
$(NH_4)_2H_2ST$
$(NH_4)_3HST$
$(NH_4)_4ST$
$(ArgH)_n(H)_{4-n}ST$, n = 1 - 4
$(LysH)_n(H)_{5-n}BT$, n = 1 - 5
$(HisH)_n(H)_{6-n}DT$, n = 1 - 6
$(NH_4)H_4BT$
$(NH_4)_2H_3BT$
$(NH_4)_3H_2BT$
$(NH_4)_4HBT$
$(NH_4)_5BT$
$K_5BT$
$Na_5BT$

Of particular interest, are the compounds $(ArgH^+)_n(H^+)_{5-n}BT$, where n = 1 - 5.

As used in this invention, antiviral activity refers to the ability of a compound to inhibit the growth of a virus in vitro or in vivo. The viruses of particular interest in the present application are HIV-1, HIV-2, herpes simplex virus type 1 (HSV-1) and type 2 (HSV-2), and simian immunodeficiency virus (SIV). Of particular importance are HIV-1 and HIV-2, particularly HIV-1. However the present compounds may also exhibit antiviral activity towards other retroviruses and even other viruses in general, such as: viruses of the herpes family, including herpes simplex viruses type 1 and 2 and two murine retroviruses (EY10 and Cas-Br-M). The compounds may also have antimicrobial properties.

Humans suffering from diseases caused by, for example, HIV can be treated by administering to the patient a pharmaceutically effective amount of one or more of the present compounds, optionally, but preferably in the presence of a pharmaceutically acceptable carrier or diluent. There may also be included pharmaceutically compatible binding agents, and/or adjuvant materials. The active materials can also be mixed with other active materials which do not impair the desired action and/or supplement the desired action. The active materials according to the present invention can be administered by any route, for example, orally, parenterally, intravenously, intradermally, subcutaneously, or topically, in liquid or solid form. For injection purposes, the medium used will be a sterile liquid. As an injection medium, it is preferred to use water which contains the stabilizing agents, solubilizing agents and/or buffers conventional in the case of injection solutions. Desirable additives include, for example, tartrate and borate buffers, ethanol, dimethyl sulfoxide, complex forming agents (for example, ethylene diamine tetraacetic acid), high molecular weight polymers (for example, liquid polyethylene oxide) for viscosity regulation or polyethylene derivatives of sorbitan anhydrides.

Solid carrier materials include, for example, starch, lactose, mannitol, methyl cellulose, talc, high dispersed silicic acid, high molecular weight fatty acids (such as stearic acid), gelatin, agar-agar, calcium phosphate, magnesium stearate, animal and vegetable fats or solid high molecular weight polymers (such as polyethylene glycols). Compositions suitable for oral administration can, if desired, contain flavoring and/or sweetening agents.

A preferred mode of administration of the compounds of this invention is orally. Accordingly, the compounds may be formulated into tablet form.

The active materials according to the present invention can be employed in dosages and amounts which are determined by conventional methods in the art of pharmacology. Thus, the materials can be used at a dosage range in humans of from 0.1 to 100 mg/kg total body weight/day. A more preferred range lies between 1 to 30 mg/kg total body weight/day. A most preferred range is 1 to 20 mg/kg total body weight/day. The dosages may be administered at once, or may be divided into a number of smaller doses to be administered at varying intervals of time.

The compounds of the present invention are expected to be useful in treating mammals, including humans. Other mammals include dogs, cats, monkeys, horses, and the like.

In order to be useful, the compounds should exhibit relatively low toxicity towards normal cells. The toxicities of some of the present compounds were measured in Vero cells and human peripheral blood

4

mononuclear cells, and toxicity values were determined. The toxicity tests are described hereinbelow.

The invention now being generally described, the same will be better understood by reference to certain specific examples which are included herein for purposes of illustration only and are not intended to be limiting of the invention or any embodiment thereof, unless specified.

## EXPERIMENTAL EXAMPLES

Synthesis of Representative Compounds

I. $H_4SiW_{12}O_{40}$ (1). Pope, Michael Thor, Heteropoly and Isopoly Oxometalates, Springer-Verlag, Berlin (1983), p. 1.

A solution of $Na_2WO_4.2H_2O$ was made by dissolving 25 g of the compound in 125 ml of deionized water with stirring in a 250 ml round-bottomed flask. Then 1.25 ml of sodium silicate solution, $NaSiO_3$ saturated in water (water glass) was added and the resulting mixture was heated to 80°C for 5 minutes. At this point, 15 ml of concentrated HCl was added over 1.5 hours, using a dropping funnel, to the hot, stirring, solution. The solution was then taken off the heat and transferred to a separatory funnel, where it was allowed to cool for 15 minutes. The addition of 10 ml HCl and 50 ml of $Et_2O$ resulted in the formation of three layers. The top two layers were clear, the top layer being ether and the next lower layer being the water layer. There was then a murky white, ill-defined border of unknown composition which was probably a mixture or that layer and the lowest layer. The lowest layer was a clear viscous fluid which was the etherate which contained the product. The layers were each drawn off and the etherate layer was allowed to sit overnight at RT and form crystals. These crystals were recrystallized from water and the white crystalline product, 14 g, was characterized by IR, UV, and $^{183}W$ NMR. These spectra were characteristic for the compound.

II. Synthesis of amino acid salts: lysinium, argininium, and histididium, from 1.

A. Lysinium salt: (2)

This procedure was adapted from the E. O. North procedure for the sodium salt. Jasmin, C., et al, J. Natl. Cancer Inst. (1974), 53, 469. A saturated solution of 1 in water was prepared using 3.6 g in a minimal amount of water. A saturated solution of lysine monohydrochloride (from Aldrich) using 1 g in a minimal amount of water was then prepared. The lysine solution was then added to the solution of 1 with stirring. The addition resulted in a bright white precipitate, whose polyoxometallate nature was qualitatively confirmed by a quick color change to blue when a small amount was exposed to a metal spatula for a few minutes. The precipitate was vacuum-filtered off and a small amount vacuum-dried at 40°C. An IR of the dried material (1:35) showed the presence of the characteristic set of peaks of 1 as well as several peaks which were due to a lysine countercation. Recrystallization of the compound was effected by dissolving the compound in water with low heat and allowing the solution to sit at room temperature until crystals formed. After several days, blue-white gelatinous clumps formed. The clumps became a chunky white powder when vacuum-filtered. Using the same recrystallization conditions, a second recrystallization produced more clumps which became white microcrystals when filtered off by a vacuum. The $^1H$ NMR of the product confirmed the presence of lysine (by comparison of the spectra to reported spectra. An IR also showed the presence of characteristic peaks for lysine.HCl as well as the reference peaks expected for the anion of 1.

B. Argininium salt: (3)

The same general procedure was used for the preparation of this salt as was used for the preparation of 2 with a substitution of arginine monohydrochloride (from Alfa) for the countercation. In contrast with the lysine salt, the white precipitate formed only after the addition of 3 ml of the saturated solution rather than immediately. Due to this slower precipitate formation, the solution was allowed to stir for 1 hour before vacuum filtration removed the product. Recrystallization was effected through treatment of 3 g of the compound with 25 ml water. While most of the compound remained undissolved after sonication and stirring at low heat, the filtrate was collected and left to sit at room temperature for several days. White crystals formed and were collected (0.5 g). The IR spectrum of the product indicated both the characteristic peaks for the anion of 1 and the peaks for the argininium countercation were observed. The $^1H$ NMR showed an almost identical peak pattern as that which was seen for the starting material of the argininium countercation as well as conforming well with the literature spectrum of its components.

C. Histidinium salt: (4)

The same general procedure was followed in this preparation as was used in the synthesis of 2 with the substitution of histidine hydrochloride (from Alfa) for the countercation source. A saturated solution of 1 was prepared by dissolving 4 g of it in a minimal amount of water. A saturated solution of histidine hydrochloride in water was prepared and was added dropwise to the solution of 1. During initial additions, brief flashes of white ppt appeared and quickly redissolved. As more of the countercation solution was added (approx. 2 ml) the ppt became permanent and upon addition of an additional ml of solution, the resulting solution was allowed to stir for an hour and the precipitate produced vacuum-filtrated off. Recrystallization was effected by treatment of 1.71 g with 200 ml water. While some of the compound remained insoluble, the filtrate was collected and allowed to sit until white microcrystals formed. The IR and $^1$H NMR of the compound were consistent for the compound's components.

III. Synthesis of $K_5BW_{12}O_{40}$ (5):

The following were added successively to 100 ml of water with vigorous stirring: 50 g of $Na_2WO_4.2H_2O$, 2.5 g $H_3BO_3$, and 30 ml 6 M HCl. The resulting solution, approx. pH 6, was allowed to boil for 8 hours. Water was added from time to time as needed. Any solid formed during this time, beta-paratungstate $(Na_{10}W_{12}O_{42}.xH_2O)$, was filtered off. The solution was then acidified to pH 2 with 6 M HCl and boiled for an additional 30 minutes. Precipitation was effected using 10 g solid KCl. After filtration and a diethyl ether wash, the crude product was recrystallized at 60°C in 25 ml water. An IR of the product was typical of literature spectra of the compound.

IV. Synthesis of amino acid salts: lysinium, argininium, and histidinium, from 5.

A. Lysinium salt (6):

The same general procedure was used for this synthesis as was used for the amino acid salts of 1. A solution of 5 was prepared by dissolving 1.3 g in 25 ml water. This produced a cloudy liquid. A saturated solution of lysine hydrochloride in water was also prepared. This solution was added dropwise to the solution of 5 with stirring. During addition, the solution gradually became clearer with each drop added and the cloudiness disappeared altogether after the addition of 20 ml. The solution was allowed to sit until white, roundish globules formed. These were filtered off and redissolved in water and allowed to sit and recrystallize. Clear polyhedras came out of solution 10 days later (0.8 g). IR and $^1$H NMR were used to characterize the compound. The IR indicated the presence of both the anion of 5 as well as the lysinium countercation. The $^1$H NMR gave better proof that the lysinium countercation was present by the comparison of the spectra with literature spectra of lysine hydrochloride and with the spectra of the starting materials.

B. Synthesis of $X_5BW_{12}O_{40}$ where X is $NH_4^+$, $Na^+$, ArginineH$^+$, and HistidineH$^+$, from $H_5BW_{12}O_{40}$ (5).

These derivatives were prepared using 5 in solution in the preparatory step before precipitation of 5 by addition of KOH. Instead of using KOH, the different salts were prepared by acidification of the 5 solution by 6M HCl to pH 1.4. The solution was then separated into 5 equal aliquots. At this point, the countercation saturated aqueous solutions were added until pH 2.3 was attained. The solutions remained undisturbed for 30 minutes and the various precipitates filtered.

1. X is ArginineH$^+$ (7):

A saturated aqueous solution of arginine hydrochloride was added as the countercation source and a yield of 3.6 g of white precipitate was produced. This was characterized by IR and $^1$H NMR, and the spectra were consistent with the compound's components.

2. X is HistidineH$^+$ (8):

A saturated aqueous solution of histidine hydrochloride was added as the countercation source and a yield of 3.7 g of white precipitate was produced. The compound was characterized by IR and $^1$H NMR, and the spectra were consistent with the compound's components.

## Biological Activity

At present, there are no available animal models for HIV-1. Chimpanzees can be infected but do not develop disease. Therefore, in general, in vitro activity towards infected cells is reported hereinbelow.

Cells. Human peripheral blood mononuclear cells (PBMC) from healthy HIV-1 seronegative and hepatitis B virus seronegative donors were isolated by Ficoll-Hypaque discontinuous gradient centrifugation at 1,000 x g for 30 minutes, washed twice in phosphate-buffered saline (pH 7.2; PBS), and pelleted at 300 x g for 10 minutes. Before infection, the cells were stimulated by phytohemagglutinin (PHA) at a concentration of 16.7 µg/ml for three days in RPMI 1640 medium supplemented with 15% heat-inactivated fetal calf serum, 1.5 mM L-glutamine, penicillin (100 U/ml), streptomycin (100 µg/ml), and 4 mM sodium bicarbonate buffer.

Viruses. HIV-1 (strain LAV-1) was obtained from Dr. P. Feorino (Centers for Disease Control, Atlanta, GA). The virus was propagated in human PBMC using RPMI 1640 medium, as described previously (McDougal, et al., J. Immun. Meth. 76:171-183, 1985) without PHA and supplemented with 7% v/v interleukin-2 (Advanced Biotechnologies, Silver Spring, MD), 7 µg/ml DEAE-dextran (Pharmacia, Uppsala, Sweden), and 370 U/ml anti-human leucocyte (alpha) interferon (ICN, Lisle, IL.). Virus obtained from cell-free culture supernatant was titrated and stored in aliquots at -80°C until use.

Inhibition of virus replication in human PBMC. Uninfected PHA-stimulated human PBMC were uniformly distributed among 25 cm$^2$ flasks to give a 5 ml suspension containing about $2 \times 10^6$ cells/ml. Suitable dilutions of virus were added to infect the cultures. The mean reverse transcriptase (RT) activity of the inocula was 50,000 decompositions per min/ml corresponding to about 100 $TCID_{50}$, as determined by Groopman et al. (Groopman J. E., et al. (1987), AIDS Res. Human Retro. 3:71-85). The drugs at twice their final concentrations in 5 ml of RPMI 1640 medium, supplemented as described above, were added to the cultures. Uninfected and untreated PBMC at equivalent cell densities were grown in parallel as controls. The cultures were maintained in a humidified 5% $CO_2$-95% air incubator at 37°C for six days after infection at which point all cultures were sampled for supernatant reverse transcriptase (RT) activity. Previous studies had indicated that maximum RT levels were obtained at that time.

RT activity assay. Six ml supernatant from each culture was clarified from cells at 300 x g for 10 minutes. Virus particles were pelleted from 5 ml samples at 40,000 rpm for 30 minutes using a Beckman 70.1 Ti rotor and suspended in 200 µl of virus disrupting buffer (50 mM Tris-Cl, pH 7.8, 800 mM NaCl, 20% glycerol, 0.5 mM phenylmethyl sulfonyl fluoride, and 0.5% Triton X-100).

The RT assay was performed in 96-well microtiter plates, as described by Spira et al. (Spira, T. J., et al. (1987), J. Clin. Microbiol. 22:97-99). The reaction mixture, which contained 50 mM Tris-Cl pH 7.8, 9 mM $MgCl_2$, 5 mM dithiothreitol (DTT), 4.7 µg/ml $(rA)_n \cdot (dT)_{12-18}$, 140 µM dATP, and 0.22 µM [3H]TTP specific activity 78.0 Ci/mmol, equivalent to 17,300 cpm/pmol; NEN Research Products, Boston, MA., was added to each well. The sample (20 µl) was added to the reaction mixture which was then incubated at 37°C for 2 hours. The reaction was terminated by the addition Of 100 µl 10% trichloroacetic acid (TCA) containing 0.45 mM sodium pyrophosphate. The acid-insoluble nucleic acids which precipitated were collected on glass filters using a Skatron semi-automatic harvester (setting 9). The filters were washed with 5% TCA and 70% ethanol, dried, and placed in scintillation vials. Four ml of scintillation fluid (Econofluor, NEN Research Products, Boston, MA) were added and the amount of radioactivity in each sample was determined using a Packard Tri-Carb liquid scintillation analyzer (model 2,000CA). The results were expressed in dpm/ml of original clarified supernatant.

Cytotoxicity assay. The drugs were evaluated for their potential toxic effects on uninfected PHA-stimulated human PBMC. Flasks were seeded so that the final cell concentration was $2 \times 10^5$ cells/ml. The cells were cultured with and without drug for 6 days at which time aliquots were counted for cell viability, as assessed by the trypan blue dye-exclusion method using a hemacytometer. The median inhibitory concentration (IC50) was calculated using the median effect method (Chou, J., and T.-C. Chou (1985), Elsevier-Bisosoft, Elsevier Science Publishers, Cambridge, U.K.; Chou, T.-C., and P. Talalay (1984), Adv. Enz. Regul. 22:27-55).

Evaluation of compounds on purified retroviral RT. HIV-1 RT was isolated from detergent disrupted virions obtained from the cell-free supernatant of infected PHA-stimulated PBMC. The enzyme was purified by passing the extract through ion-exchange chromatography columns, as described previously (Eriksson, B., et al. (1977), Antimicrob. Agents Chemother. 31: 600-604). Briefly, 750 ml of culture fluid harvested from HIV-1-infected PBMC were ultracentrifuged to pellet the virus. The pellet was dissolved in buffer A [50 mM Tris-HCl, pH 7.9/0.25% Nonidet P-40/5 mM dithiothreitol/20% (vol/vol) glycerol] containing 1 mM EDTA, and freeze-thawed four times. After clarification by centrifugation, the enzyme was partially purified from the supernatant by passing the extract through a DEAE-cellulose column (Whatman DE-52; 2.6 x 15 cm) previously equilibrated with buffer B (50 mM Tris-Cl, pH 7.9, 50 mM KCl, 1 mM EDTA, 1 mM dithiothreitol, 20% glycerol). Elution was performed using a linear gradient 50-500 mM KCl in buffer B. Fractions containing enzyme activity were dialyzed against buffer B and were further purified on a phosphocellulose column (Whatman P-11) using a linear KCl (50-500 mM) gradient in buffer B. The peak fractions were pooled and dialyzed against buffer B. The enzyme was characterized as HIV-1 reverse transcriptase based on its cation, salt, pH, and template requirements according to previous descriptions (Eriksson, B., et al. (1977), Antimicrob. Agents Chemother. 31: 600-604). The standard reaction mixture was used to evaluate the inhibitory effect of the drugs, as previously described (Eriksson, B., et al. (1977), Antimicrob. Agents Chemother. 31: 600-604). Briefly, the reaction mixtures (final volume 100 µl) contained 100 mM Tris-HCl, pH 8.0, 50 mM KCl, 2 mM $MgCl_2$, 5 mM DTT, 400 µg/ml bovine serum albumin, 3 µg/ml $(rA) \cdot (dT)_{12-18}$ and 1 µM of [3H]TTP (specific activity 82.3 Ci/mmol). The reactions were started by the addition of 10 µl of partially purified RT, incubated at 37°C for 60 min., and processed as previously described (Eriksson, B., et al. (1977), Antimicrob. Agents Chemother. 31: 600-604).

Cell culture assays for herpes simplex viruses. Mycoplasma-free Vero cells, obtained from Flow Laboratory (McLean, VA), were used for the plaque assays. The methodologies for the plaque reduction and cytotoxicity assays have been previously described (Schinazi, R. F., et al. (1982), Antimicrob. Agents Chemother. 22:499-507).

Median-effect method. Dose-effect relationships were analyzed by the median-effect equation (Chou, T.-C., and P. Talalay (1984), Adv. Enz. Regul. 22:27-55). The slope (m) and median effective and inhibitory concentration (EC50 and $IC_{50}$) values were obtained by using a computer program developed Chou and Chou (Chou, J., and T.-C. Chou (1985), Elsevier-Biosoft, Elsevier Science Publishers, Cambridge, U.K.).


## UV/XC Assay for Murine Retroviruses


Cells. Mouse embryo fibroblasts (SC-1) and XC cells were obtained from the American Type Culture Collection, Rockville, MD. The XC cell line was derived from a transplantable tumor induced in newborn White-Weston rats by the intramuscular injection of the Prague strain of Rous sarcoma virus. This cell line does not release infectious virus. The cells were maintained in Eagle's minimum essential medium with Hanks balanced salt solution, supplemented with penicillin (100 U/ml), streptomycin (100 µg/ml), sodium bicarbonate (24 mM), Hepes (25 mM), and either 2 (maintenance) or 10 (growth) percent inactivated newborn or fetal calf serum.

Virus quantitation by UV-XC assay. The XC test (Rowe, W. P., W. E. Pugh, and J. W. Hartley. 1970. Plaque assay techniques for murine leukemia viruses. Virology 42:1136-1139) is a plaque-type of assay which tests for the presence and titer of B- and N-tropic murine leukemia viruses. Because these viruses produce no overt signs of infection of SC-1 mouse cells, it is necessary to overlay the virus producing culture with an indicator cell line, XC. The number of syncytia that are produced is a direct measure of the virus titer in FFU.

Six-well plates were seeded with $1 \times 10^5$ SC-1 mouse cells on the first day. On the following day the medium was removed and the inoculum (0.2 ml) was added in medium containing polybrene (4 µg/ml). For determining the antiviral activity of the compounds in culture, the virus dilution selected produced in untreated cultures about 80 foci per well. After 1 hour, the virus was removed and various concentrations of the drug were added. The plates were replenished with medium on the third and fifth days. The medium was removed on the eighth day and the cells exposed to UV irradiation (60 erg/mm$^2$ per sec) for 30 seconds. Medium containing $10^6$ XC cells was then added to each plate. On the eleventh day, the plates were fixed with 10% buffered Formalin and stained with crystal violet. Comparison of the number of foci to those found in virus-infected cells receiving no drug allowed determination of whether a drug at a particular concentration was effective. Control cells without virus but receiving the drug provided an estimate of the drug's toxic effects as quantitated by using trypan blue staining.


Bone marrow toxicity assays

Preparation of Cells. Human bone marrow cells were collected by aspiration from the posterior iliac crest of normal healthy volunteers, treated with heparin and the mononuclear population separated by Ficoll-Hypaque gradient centrifugation. Cells were washed twice in Hanks balanced salt solution, counted with a hemacytometer and their viability was > 98% as assessed by trypan blue exlcusion.

Assay of Colony-Forming Unit Granulocyte-Macrophage (CFU-GM) for Drug Cytotoxicity. The culture assay for CFU-GM was performed using a bilayer soft-agar method as recently described (Sommadossi and Carlisle. 1987, Antimicrob. Agents Chemother. 31:452-454). McCoy 5A nutrient medium supplemented with 15% dialyzed fetal bovine serum (heat inactivated at 56°C for 30 min.) (Gibco Laboratories, Grand Island, NY) was used in all experiments. This medium was completely lacking in thymidine and uridine. Cells were cloned in 0.3% agar in the presence of increasing concentrations of the modulating compound or in medium alone (control). After 14 days of incubation at 37°C in a humidified atmosphere of 5% $CO_2$ in air, colonies ($\geq 50$ cells) were counted using an inverted microscope.


Bone marrow toxicity studies:

The toxicities of a variety of polyoxometallates with respect to human bone marrow cells (human granulocyte-macrophage precursor cells) were assessed. The toxicities and trends in toxicity in these tests (results given below) were not the same as those displayed in mice toxicity studies. The bone marrow toxicity assays revealed some interesting and most encouraging results:

1) The ammonium salts proved to be substantially less toxic than the free acid forms of any given polyoxometallate,

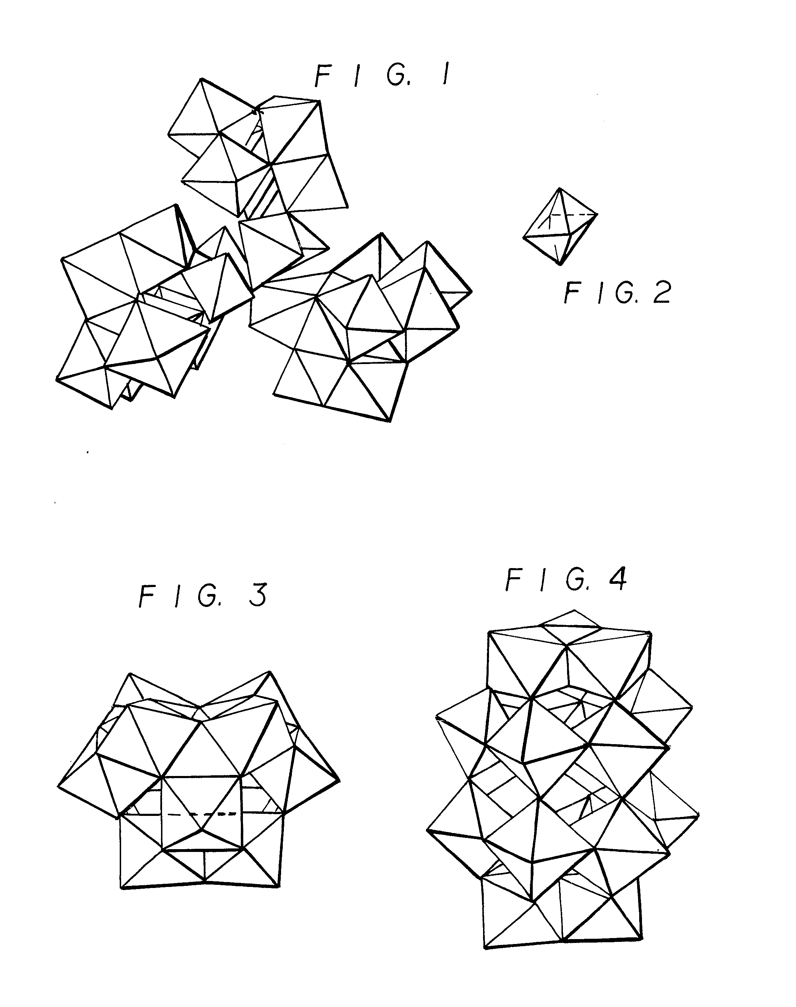

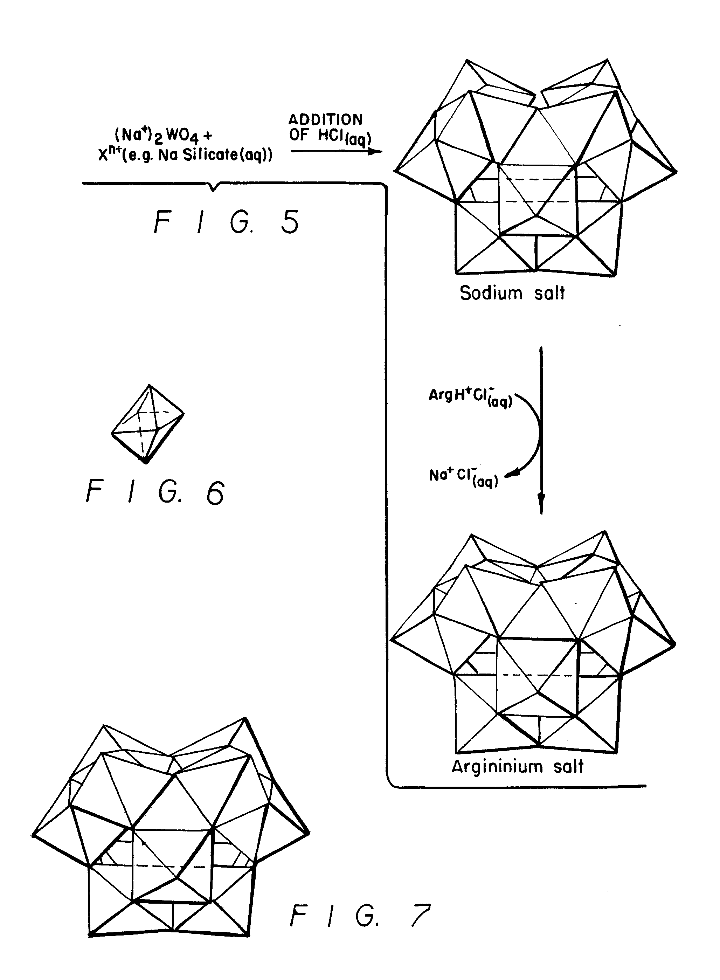

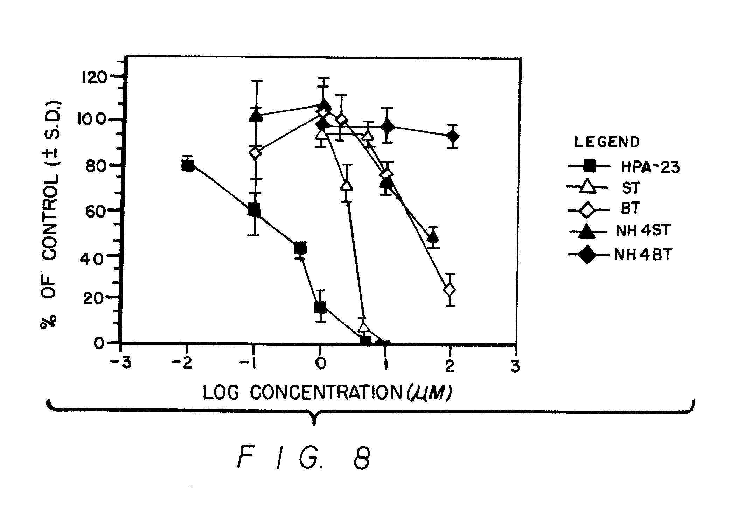

2) The following order of toxicity in these assays was established: ammonium BT = NH₄BT (least toxic) < BT and NH₄ST < AZT and ST < HPA-23, the French polyoxometallate anti-HIV-1 agent currently in clinical trial (most toxic) (Fig. 3).

Ammonium BT displayed effectively no toxicity to these human bone marrow cells.

Replacing the inorganic cations of the polyoxometallates (Na⁺, K⁺, and H⁺, etc.) with ammonium and basic amino acid derived cations, does not adversely affect the otherwise high antiviral efficacy and low toxicity of the polyoxometallates shown in cell culture (PBMC), but the latter polyoxometallate salts show greatly improved toxicity to human bone marrow cells and markedly improved tolerance in mammals in general relative to the former salts.

Results of activity testing on some of the present compounds are shown in Table 1 herein.

EP 0 360 619 A2

| Compound Formula | Mol. Weight | Cell-Free RT (virion-derived) | HIV-1 (LAV-1) | HSV-1 | HSV-2 | Friend, Spleen (EY-10) | Mouse Ecotropic, CNS (CAS-Br-M) | SIV |
|---|---|---|---|---|---|---|---|---|
| AZT (Reference Compound) | 267 | | $3.08 \times 10^{-3}$ | >100 | >100 | 0.01 | $7.3 \times 10^{-3}$ | $1.27 \times 10^{-3}$ $2.88 \times 10^{-3}$ |
| $\alpha$ H$_3$PT | 2,988 (6 H$_2$O) | >100 | 13.98 | 12.7 | 18.7 | | | |
| $\alpha$ and $\beta$ H$_4$ST | 3,004 (7 H$_2$O) | 26.1 | 0.12, 0.21 | 1.6 | 1.5 | 0.17 | | |
| $\alpha$ and $\beta$ H$_5$BT | 3,042 (10 H$_2$O) | 2.6 | 0.46, 0.11, 0.44 | <1 | | 0.18 | | |
| $\varepsilon$ H$_4$ST | 3,004 (7 H$_2$O) | 34.6 | 0.25 | 3.16 | 1.1 | 0.29 | | |
| H$_6$DT | 4,763 (15 H$_2$O) | 0.14 | 0.52 | 0.16 | 0.18 | | | |
| $\alpha$ (NH$_4$)$_6$DT | 4,865 (15 H$_2$O) | 0.13 | 0.91 | 0.33 | 0.24 | | | |
| $\alpha$ (NH$_4$)$_n$H$_{(4-n)}$ST | 3,111 (n=1, 10 H$_2$O) | 17.3 | 2.4 (2.7, 2.26) | | | 0.18, 0.88 | 0.79 | 0.52 (0.042, 4.33, 1.01) |
| $\alpha$ (NH$_4$)$_n$H$_{(5-n)}$BT | 3,110 (n=1, 10 H$_2$O) | 3.3 | 3.2 (3.17) | | | 0.49, 0.90 (0.70) | 1.16 | 2.3 (0.92, 1.42) |
| $\alpha$-K$_5$BT | 3,233 (10 H$_2$O) | 2.9 | 0.36 | | | 0.19, 0.80 | 0.91 | |
| (NH$_4$)$_5$BT | 2,947 | 68.1 | 6.69, 11.6 | >10 | >10 | | | |
| Na$_5$BT | 2,972 | 32.2 | 3.61, 10.9 | >10 | >10 | | | |
| (NH$_4$)$_4$W$_{10}$ | 2,423 | 24.2 | 2.00, 0.96 | >10 | 9.7 | 0.58 | | |
| (Me$_4$N)$_4$W$_{10}$ | 2,648 | 10.1 | 3.05, 3.30 | 5.98 | 8.78 | | | |
| (HISH$^+$)$_n$H$^+_{(5-n)}$BT | 3,632 | 30.5 | 12 | >10 | >10 | | | |
| (LYSH$^+$)$_n$H$^+_{(5-n)}$BT | 3,587 | 18.8 | 3.00, 4.3 | 6.69 | 2.78 | | | |
| (ARGH$^+$)$_n$H$^+_{(5-n)}$BT | 3,727 | 25.4 | 0.58, 1.70 | 1.35 | 3.16 | | | |
| (HISH$^+$)$_n$H$^+_{(4-n)}$ST | 3,494 | 22.3 | 0.22, 0.65 | 0.95 | 0.87 | 0.92 | | |
| (LYSH$^+$)$_n$H$^+_{(4-n)}$ST | 3,458 | 20.5 | 1.61, 0.68 | 0.32 | <1 | 0.95 | | |
| (ARGH$^+$)$_n$H$^+_{(4-n)}$ST | 3,570 | 14.2 | 1.03, 0.77 | 0.18 | <1 | 0.99 | | |
| HPA-23 (Reference Compound) | | | 0.29 | | | | | |

| | IC50 (μM) | |
| | | |
| Compound Formula | Toxicity >100 μM = Nontoxic PBM Cells | Toxicity >100 μM = Nontoxic Vero Cells |
| --- | --- | --- |
| AZT, Reference Compound | >100 | 29.0 μM |
| α $H_3PT$ | | 10 μM |
| α and β $H_4ST$ | | 80 μM |
| α and β $H_5BT$ | 76%, 200 μM | 85 μM |
| β $H_4ST$ | >100 μM | 8 μM |
| $H_6DT$ | 90%, 100 μm | 81.9%, 100 μM |
| α $(NH_4)_6DT$ | 76%, 100 μM | 83%, 100 μM |
| α $(NH_4)_nH_{(4-n)}ST$ | >100 μM | ~50 μM |
| α $(NH_4)_nH_{(5-n)}BT$ | >100 μM | ~50 μM |
| α-$K_5BT$ | | ~80 μM |
| $(NH_4)_5BT$ | | >100 μM |
| $Na_5BT$ | | >100 μM |
| $(NH_4)_4W_{10}$ | >100 μM | >100 μM |
| $(Me_4N)_4W_{10}$ | | >100 μM |
| $(HISH^+)_nH^+_{(5-n)}BT$ | | >9.9 μM |
| $(LYSH^+)_nH^+_{(5-n)}BT$ | >100 μM | >100 μM |
| $(ARGH^+)_nH^+_{(5-n)}BT$ | 328 μM | >100 μM |
| $(HISH^+)_nH^+_{(4-n)}ST$ | 181.5 μM | >100 μM |
| $(LYSH^+)_nH^+_{(4-n)}ST$ | 2,447 μM | >100 μM |
| $(ARGH^+)_nH^+_{(4-n)}ST$ | >100 μM | >100 μM |
| HPA-23 (Reference Compound) | | |

## Assays in Whole Infected Mice

Tests were conducted in whole infected mice using one of the compounds of the present invention. The methodology of this test is the same as was used in Ruprecht et al, Nature 323:467-469, except that Friend virus was used instead of Rauscher virus. The results of these tests are shown in Table 2 hereinbelow.

Table 2

Splenomegaly Model in BALB/c Mice

| Treatment | Dose | | Units | Administration | N | % Mortality | MDD | Mean Spleen wt. (mg) ± SD | % Effectiveness | Day of Sacrifice |
|---|---|---|---|---|---|---|---|---|---|---|
| PBS | 0 | mkd | | IP QD x 10 | 10 | 0 | | 2,028 ± 508 | 0 | 16 |
| $(ArgH^+)_4H^+BT$ | 10 | mkd | | IP QD x 10 | 10 | 0 | | 2,287 ± 864 | -12.8 | 16 |
| $(ArgH^+)_4H^+BT$ | 30 | mkd | | IP QD x 10 | 10 | 0 | | 2,130 ± 693 | -5.0 | 16 |
| $(ArgH^+)_4H^+BT$ | 60 | mkd | | IP QD x 6 | 10 | 20 | 14.0 ± 0 | 644 ± 432 | 68.2 | 16 |
| AZT | 30 | mkd | | IP QD x 10 | 10 | 0 | | 1,249 ± 1,152 | 38.4 | 16 |
| AZT | 60 | mkd | | IP QD x 10 | 10 | 0 | | 988 ± 836 | 51.3 | 16 |

Abbreviations:

MDD: Mean day of death.

IP: Intraperitonally.

QD x 10: Once/day for 10 days.

QD x 6: Once/day for 6 days.

N: Number animals/jp.

PBS: Phosphate buffered saline.

mkd: mg/kg/d

EP 0 360 619 A2

Obviously, numerous modifications and variations of the present invention are possible in light of the above teachings. It is therefore to be understood that within the scope of the appended claims, the invention may be practiced otherwise than as specifically described herein.

## Claims

1. A pharmaceutical composition for the treatment of an infection by a retrovirus of a mammal, characterised by a content of a compound having a molecular formula selected from the group consisting of:

$(X^+)_n(H^+)_{4-n}(SiW_{12}O_{40})^{4-}$   $n = O - 4,$
$(X^+)_m(H^+)_{5-m}(BW_{12}O_{40})^{5-}$   $m = O - 5,$
$(X^+)_n(H^+)_{4-n}(W_{10}O_{32})^{4-}$ n   $= 0 - 4$
$(X^+)_p(H^+)_{6-p}(P_2W_{18}O_{62})^{6-}$   $p = O - 6$

wherein X is K, Na, $NH_4$, a monocationic naturally-occurring amino acid or a polypeptide made up of naturally-occurring basic amino acids and which contains one amino acid selected from the group consisting of arginine, lysine and histidine in protonated form, in combination or association with a pharmaceutically acceptable carrier, diluent or excipient.

2. A composition according to Claim 1, wherein said compound is selected from the group consisting of:

$(ArgH^+)_n(H^+)_{5-n}BT$, where n = 1 - 5.

3. A composition according to Claim 2, wherein said compound is $(ArgH^+)_n(H^+)_{5-n}BT$, where n = 3 - 5.

4. A composition according to Claim 1, 2 or 3, wherein said monocationic species is selected from the group consisting of $K^+, Na^+, NH4^+, NR4^+$ wherein R is $C_{1-5}$ alkyl, arginine $H^+$, lysine $H^+$ and histidine $H^+$.

5. A composition according to any one of the preceding claims, in a form suitable for oral or parenteral administration.

6. A composition according to any one of the preceding claims, in unit dosage form.

7. A composition according to claim 6, wherein the unit dose is in the range 0.1 to 100 mg/Kg of body weight.

8. The use of a compound having a molecular formula selected from the group consisting of :

$(X^+)_n(H^+)_{4-n}(SiW_{12}O_{40})^{4-}$ n = O - 4,
$(X^+)_m(H^+)_{5-m}(BW_{12}O_{40})^{5-}$ m O - 5,
$(X^+)_n(H^+)_{4-n}(W_{10}O_{32})^{4-}$ n = 0 - 4
$(X^+)_p(H^+)_{6-p}(P_2W_{18}O_{62})^{6-}$ p = O - 6

wherein X is K, Na, $NH_4$, a monocationic naturally-occurring amino acid or a polypeptide made up of naturally-occurring basic amino acids and which contains one amino acid selected from the group consisting of arginine, lysine and histidine in protonated form, to prepare a drug for the treatment of patients infected with a retrovirus.

9. The use according to claim 8, wherein the retrovirus is HIV.

10. A compound selected from the group consisting of:

(Basic Amino acid $H^+)_n(H^+)_{5-n}$ BT, where n = 1 - 5
(Basic Amino acid $H^+)_n(H^+)_{5-n}$ ST, where n = 1 - 4.

11. A compound according to claim 10, wherein the basic amino acid is selected from the group consisting of arginine, lysine and histidine.

F I G. I

F I G. 2

F I G. 3

F I G. 4

$(Na^+)_2 WO_4 +$
$X^{n+}$(e.g. Na Silicate(aq))

ADDITION
OF $HCl_{(aq)}$

Sodium salt

$F \ I \ G. \ 5$

$F \ I \ G. \ 6$

$Arg H^+ Cl^-_{(aq)}$

$Na^+ Cl^-_{(aq)}$

Argininium salt

$F \ I \ G. \ 7$

F I G. 8